# FASCICULE DE BREVET EUROPEEN

(11) **EP 2 094 386 B1**
(45) Date de publication et mention de la délivrance du brevet: **30.01.2019**
(21) Numéro de dépôt: 07871990.3
(22) Date de dépôt: 20.12.2007
(51) Int. Cl.: B01J 23/00, B01J 23/882, B01J 27/04, B01J 27/051, B01J 37/20, C01G 45/00, C07C 319/26, C07C 321/14, C10G 45/08, C01G 51/04, C01G 53/04

(54) **AGENT DE SULFURATION DE CATALYSEUR D'HYDROTRAITEMENT ET SON UTILISATION POUR LA PRESULFURATION IN-SITU ET EX-SITU**
SULFIDIERUNGSMITTEL FÜR EINEN HYDRIERUNGSKATALYSATOR UND VERWENDUNG FÜR IN-SITU- UND EX-SITU-VORSULFIDIERUNG
SULPHIDATION AGENT FOR HYDROPROCESSING CATALYST AND USE THEREOF FOR IN-SITU AND EX-SITU PRESULPHIDATION

(30) Priorité: 22.12.2006 FR 0655884; 21.05.2007 US 939152 P; 03.09.2007 FR 0757330
(43) Date de publication de la demande: 02.09.2009
(73) Titulaire: ARKEMA FRANCE, 92700 Colombes (FR)
(72) Inventeur: HUMBLOT, Francis, F-64300 Lanneplaa (FR); SCHMITT, Paul Guillaume, 64230 Lescar (FR); FREMY, Georges, F-64390 Sauveterre de Bearn (FR)
(86) Numéro de dépôt international: PCT/FR2007/052579
(87) Numéro de publication internationale: WO 2008/087330

(56) Documents cités:
- EP-A- 0 976 726
- EP-A2- 0 276 681
- JP-A- 5 331 047
- US-A- 2 993 938
- US-A- 5 820 749
- T. HIRABAYASHI, S. MOHMAND, H. BOCK: "Gasphasen-Reaktionen, 23: Thermische Zersetzung offenkettiger Dialkyl-sulfide, -disulfide und -diselenide" CHEM. BER., vol. 115, 1982, pages 483-491, XP002448908 Weinheim

## Description

La présente invention concerne le domaine de l'hydrotraitement de charges hydrocarbonées et a plus particulièrement pour objet un procédé de présulfuration des catalyseurs utilisés à cet effet.

Les catalyseurs d'hydrotraitement de charges hydrocarbonées, concernés par la présente invention, sont utilisés dans des conditions appropriées pour convertir en présence d'hydrogène les composés organosoufrés en hydrogène sulfuré, opération que l'on appelle hydrodésulfuration (HDS), et pour convertir les composés organoazotés en ammoniac dans une opération que l'on appelle hydrodéazotation (HDN).

Ces catalyseurs sont généralement à base de métaux des groupes VI B et VIII de la classification périodique des éléments, tels que le molybdène, le tungstène, le nickel et le cobalt. Les catalyseurs d'hydrotraitement les plus courants sont formulés à partir des systèmes cobalt-molybdène (Co-Mo), nickel-molybdène (Ni-Mo) et nickel-tungstène (Ni-W), déposés sur des supports minéraux poreux tels que des alumines, des silices, des silices-alumines. Ces catalyseurs fabriqués industriellement à des tonnages très importants sont fournis à l'utilisateur sous leurs formes oxydes (par exemple, les catalyseurs oxyde de cobalt-oxyde de molybdène sur alumine symbolisés par l'abréviation : Co-Mo/alumine).

Cependant, ces catalyseurs ne sont actifs dans les opérations d'hydrotraitement que sous forme de sulfures métalliques. C'est pourquoi, avant d'être utilisés, ils doivent être sulfurés.

Concernant l'activation des catalyseurs d'hydrotraitement, la sulfuration de ces catalyseurs est une étape importante pour obtenir le maximum de leurs performances en HDS et en HDN. Comme l'indiquent les auteurs de Hydrotreating Catalysis (Catalysis, vol. 11, 1996, p. 25, édité par J.R. Anderson et M. Boudart), l'expérience pratique a montré que la procédure de sulfuration peut avoir une influence significative sur l'activité et la stabilité du catalyseur, et beaucoup d'efforts ont été consacrés à améliorer les procédures de sulfuration.

La méthode de sulfuration d'un catalyseur la plus directe consiste à traiter ce dernier par l'hydrogène sulfuré mélangé à de l'hydrogène. Cependant, cette méthode qui a fait l'objet de nombreux brevets (US 3.016.347, US 3.140.994, GB 1.309.457, US 3.732.155, US 4.098.682, US 4.132.632, US 4.172.027, US 4.176.087, US 4.334.982, FR 2.476.118) présente des inconvénients majeurs (toxicité aiguë, difficulté d'approvisionnement en H₂S) ne permettant pas sa mise en oeuvre sur tous les sites industriels.

Les procédures industrielles de sulfuration des catalyseurs sont généralement effectuées sous pression d'hydrogène avec des charges liquides contenant déjà des composés soufrés comme agents sulfurants. La méthode principalement utilisée dans le passé par les raffineurs consistait à sulfurer les catalyseurs avec les charges pétrolières soufrées, mais cette technique présentait des inconvénients importants à cause de la difficulté de transformer les composés soufrés en hydrogène sulfuré. Pour éviter la réduction des catalyseurs par l'hydrogène, les sulfurations, démarrées à basse température, devaient être menées lentement pour obtenir une sulfuration complète des catalyseurs à température élevée.

Des additifs soufrés ont été proposés pour améliorer la sulfuration des catalyseurs. La méthode consiste à incorporer un composé sulfuré (spiking agent) à une charge telle qu'un naphta ou à une coupe particulière telle qu'un VGO (vacuum gas oil) ou un LGO (light gas oil).

Dans US 3.140.994 a été revendiqué pour la première fois l'emploi de composés soufrés sous forme non oxydée, et notamment : disulfure de carbone, thiophène, mercaptans, sulfures organiques, notamment dialkyldisulfures, diaryldisulfures, le diméthylsulfure (DMDS) étant exemplifié.

EP 64.429 décrit une méthode performante de sulfuration au moyen d'une charge de sulfuration constituée par un mélange d'au moins un composé sulfuré et d'une charge hydrocarbonée et d'un profil de température spécifique ; parmi les composés soufrés sont cités le sulfure de carbone, les mercaptans, les composés thiophéniques, (di)sulfures, l'hydrogène sulfuré, le diméthyldisulfure (DMDS) étant particulièrement préféré pour la sulfuration des catalyseurs et avec le diméthyldisulfure est décrite dans le brevet.

H. Hallie (Oil and Gas Journal, Dec. 20, 1982, pp 69 - 74) a fait le point sur ces procédures de sulfuration sous hydrogène qui sont effectuées directement dans les réacteurs d'hydrotraitement. Ces différentes techniques de sulfuration de catalyseurs, dites "in-situ", ont été comparées et les travaux ont montré que la sulfuration avec une charge liquide additionnée d'un agent sulfurant (spiked feedstock) ayant la propriété de se décomposer à basse température est la meilleure technique de sulfuration. La technique sans agent sulfurant additionnel (nonspiked feedstock) donne un catalyseur sulfuré moins actif. L'agent sulfurant qu'on préfère additionner à la charge est le diméthyldisulfure.

Le document US 5 820 749 décrit un procédé catalytique d'hydrogénation d'hydrocarbures insaturés connu aussi sous le nome d'hydrotraitement Le catalyseur est activé avec un agent de sulfuration en présence d'hydrogène. L'agent de sulfuration peut être le méthyl disulfure, l'éthyl disulfure, le propyl disulfure ou le butyl disulfure sans préciser la pureté d'agents sulfurants mentionnés.

Il est connu de l'homme de l'art que les dialkyldisulfures peuvent être utilisés comme agent de sulfuration, cependant seul le diméthyldisulfure a été explicitement cité comme agent de sulfuration. Le diméthyldisulfure étant d'ailleurs l'agent de sulfuration de référence dans l'industrie à ce jour.

Des polysulfures organiques ont aussi été préconisés comme agents sulfurants pour la sulfuration des catalyseurs. US 4 725 569 décrit l'utilisation d'un polysulfure organique de type RSₓR' (R et R' étant des groupements alkyle en C1 - C20 pouvant être identiques ou différents, avec x compris entre 2 et 8, le DMDS étant exclu) qui consiste à imprégner à température ambiante le catalyseur par une solution contenant le polysulfure, à éliminer ensuite le solvant inerte, et enfin à effectuer la sulfuration sous hydrogène du catalyseur chargé dans le réacteur d'hydrotraitement.

Dans EP 298.111 est décrit un procédé de sulfuration de catalyseur par passage simultané d'hydrogène et d'une charge hydrocarbonée contenant un agent sulfurant de formule RSₙR' (R et R' étant des radicaux alkyles en C1 - C4 pouvant être identiques ou différents, avec n compris entre 3 et 10).

WO 01/96499 décrit l'utilisation des mélanges de disulfures issus d'une unité de désulfuration de LPG (gaz de pétrole liquéfié) d'une unité de raffinage de pétrole auxquels ont été enlevés les composés caustiques et sodiques comme agent de sulfuration. Ces mélanges de disulfures consistent en général à plus de 98 % en diméthyldisulfure, diéthyldisulfure et éthylméthyldisulfure.

EP 0976726 décrit une composition à base de DMDS à odeur masquée contenant jusqu'à 1 % en poids d'un agent masquant d'odeur choisi parmi la vanilline, l'éthylvanilline et certains esters. Ce masquage n'est effectif si la teneur en impuretés dans le DMDS est limitée, typiquement moins de 500 ppm de méthylmercaptan, moins de 1 % de diméthylsulfure.

Il est connu par l'homme de l'art que les polyalkylsulfures RSₓR' (x étant le rang moyen en soufre et x ≥ 3) se décomposent à température plus basse que les dialkylsulfures tels que le DMDS, ce qui présente l'avantage de permettre une sulfuration plus rapide des catalyseurs, avantage mis à profit dans l'industrie. Cependant l'inconvénient majeur des polysulfures, également bien connu dans l'industrie, est la formation de soufre solide et/ou de dépôt solide qui est généré lors du traitement thermique d'activation du catalyseur ; le soufre solide et/ou le dépôt solide peuvent se déposer dans les différents éléments de la raffinerie et ainsi créer des bouchages très néfastes pour la conduite de l'unité industrielle. Bien que les polysulfures se décomposent à plus basse température que les alkyldisulfures, la formation de dépôt et/ou de soufre solide lié à leur utilisation représente un problème pour les raffineurs qui leur préfère le diméthyldisulfure qui reste l'agent de sulfuration de référence dans l'industrie.

De nouvelles techniques de sulfuration des catalyseurs comprenant deux étapes ont été récemment proposées. Dans une première étape, dite "ex-situ", le catalyseur est préactivé en l'absence d'hydrogène à l'extérieur de la raffinerie après avoir été imprégné d'un agent sulfurant.

EP 130.850 décrit un procédé de présulfuration ex-situ de catalyseur consistant à traiter ledit catalyseur à l'aide d'au moins un agent de sulfuration de formule type RSₙR' (R et R' étant des radicaux organiques en C1 - C150 (alkyle, naphténique, aryle, alkylaryle, arylalkyle) pouvant être identiques ou différents, avec n compris entre 3 et 20) utilisé en solution dans un solvant ; le catalyseur sous forme oxyde est imprégné avec une solution de polysulfures organiques, (par exemple TPS 37 ou TNPS commercialisés par ARKEMA), de préférence dans un hydrocarbure de type white-spirit. Cette étape préliminaire d'incorporation au catalyseur d'un composé soufré de nature particulière est complétée par un traitement thermique du catalyseur en l'absence d'hydrogène à des températures ne dépassant pas 150 °C. Cette opération a pour effet d'éliminer le solvant organique et d'assurer la fixation du soufre au catalyseur par l'intermédiaire des polysulfures organiques. A ce stade de présulfuration, le catalyseur est stable à l'air et peut être manipulé sans précaution particulière. Il est fourni dans cet état à l'utilisateur qui, après chargement dans le réacteur d'hydrotraitement, peut achever la sulfuration du catalyseur sous hydrogène pour la transformation totale des métaux en sulfures métalliques dans le réacteur d'hydrotraitement en présence d'hydrogène.. Les techniques "ex-situ" actuellement développées à l'échelle industrielle utilisent comme produits soufrés des polysulfures organiques ou du soufre.

D'autres composés organiques polysulfurés, de différentes structures, ont été aussi proposés pour la présulfuration des catalyseurs en "ex-situ". Les produits préconisés dans FR 2.627.104 et EP 329.499 ont pour formule générale: R'-(S_{y}-R-Sₓ-R-S_{y})-R' et sont obtenus à partir d'oléfines et de chlorure de soufre par une série d'étapes successives qui font intervenir une réaction avec un monohalogénure organique suivie d'une réaction avec un polysulfure alcalin. Dans EP 338.897, les produits revendiqués sont synthétisés à partir d'oléfines et de chlorure de soufre avec une réaction complémentaire avec un mercaptide alcalin ou un mercaptate polysulfure alcalin.

La présente invention concerne un nouvel agent de sulfuration ayant l'avantage non seulement de se décomposer à plus basse température que le diméthyldisulfure, considéré comme l'agent de sulfuration de référence de l'industrie, permettant une sulfuration plus rapide du catalyseur mais aussi de limiter très significativement les dépôts solides, notamment de soufre, par rapport aux polysulfures.

L'agent de sulfuration selon l'invention consiste essentiellement en diéthyldisulfure (DEDS) ou en dipropyldisulfure(s) (DPDS) ou dibutyldisulfure(s) (DBDS) selon la revendication 1; il présente l'avantage de se décomposer à plus basse température que le diméthyldisulfure mais aussi de ne pas former de dépôt solide, notamment lié au soufre, à la différence des polysulfures.

Au sens de la présente invention, l'expression « consiste essentiellement en » signifie contient moins de 20.000 ppm d'impuretés, de préférence moins de 10.000 ppm d'impuretés, et avantageusement moins de 5.000 ppm d'impuretés.Par impuretés, on entend des traces d'un ou plusieurs sulfures que l'on peut représenter par la formule RSnR' avec n représentant le rang moyen en soufre allant de 1 à 10, et R et R' représentant H ou un chaîne alkyle, cycloalkyle, aryle, linéaire ou ramifiée en C₁-C₂₄, tels que par exemple le DMDS, le diméthyl sulfure, le methyl mercaptan, l'éthyl mercaptan, DPDS et/ou DBDS lorsque l'agent de sulfuration consiste essentiellement en DEDS.

Par dipropyldisulfure(s) (DPDS) et dibutyldisulfure(s), on entend le ou les isomères de dipropyldisulfure et respectivement de dibutyldisulfure, par exemple n-propyl et/ou iso-propyldisulfure, n- iso- et/ou ter-butyl disulfure.

Selon un mode de réalisation préféré, l'agent de sulfuration selon l'invention comprend en outre au moins une base parfumante et/ou un agent masquant d'odeurs choisi par exemple parmi les masquants d'odeur, seuls ou en mélange décrits dans EP 0976726, tels que notamment la vanilline, l'éthylvanilline, les esters de formule R¹CO₂R² dans laquelle R¹ représente un radical hydrocarboné, linéaire ou ramifié, contenant de 1 à 4 atomes de carbone, éventuellement insaturé, et R² représente un radical hydrocarboné, linéaire, ramifié ou cyclique, contenant de 2 à 8 atomes de carbone, éventuellement insaturé.

En général, la teneur en agent(s) masquant(s) et/ou base parfumante est inférieure ou égale à 1 % en poids du poids total de l'agent de sulfuration, typiquement de 0,1 à 0,5 % en poids.

Par rapport à l'activité des catalyseurs d'hydrotraitement sulfuré avec du DMDS, de manière surprenante, l'activité des catalyseurs d'hydrotraitement sulfuré avec un agent de sulfuration selon l'invention est significativement améliorée.

L'agent de sulfuration selon l'invention peut notamment être utilisé pour présulfurer « in-situ » les catalyseurs d'hydrotraitement renfermant un support à base d'au moins un oxyde d'un métal ou d'un métalloïde et au moins un métal actif; dans ce cas, il peut, par exemple être introduit en mélange avec un gazole, sous pression d'hydrogène qui peut aller de la pression atmosphérique jusqu'à 20 MPa, mais est de préférence comprise entre 1 et 5 MPa, domaine de pression couramment utilisé industriellement. Cette étape s'effectue à une température qui peut aller jusqu'à 350 °C (une température plus élevée permet de réduire la durée de sulfuration, mais augmente très significativement les risques de cokage).

Il est avantageux de mener cette étape en deux stades :
- une sulfuration primaire effectuée à une température allant de 150 à 250 °C, de préférence de 210 à 230 °C de façon à minimiser le temps nécessaire à l'obtention de la percée d'H₂S dans les gaz de sortie sans risquer une réduction prématurée, puis
- une sulfuration secondaire effectuée à une température allant de 250 à 350 °C, de préférence de 290 et 330 °C, et d'une durée suffisante pour avoir une concentration constante en H₂S dans les gaz de sortie.

La couverture d'hydrogène exprimée par le rapport du débit volumique d'hydrogène en litres normaux sur le débit volumique du gazole en litres est en général comprise entre 50 et 500 N1/1, de préférence entre 100 et 300 NL/L.

La vitesse volumique horaire (VVH), définie comme le rapport entre le débit volumique horaire de gazole et le volume de catalyseur, peut aller de 0,1 à 5 h⁻¹ et est de préférence comprise entre 1 et 3 h⁻¹, intervalle couramment utilisé industriellement.

La quantité totale de soufre apportée par le nouvel agent de sulfuration de l'invention peut en général aller de 100 à 250 % du poids de soufre stoechiométriquement requis pour la transformation totale en sulfures des oxydes du catalyseur.

L'agent de sulfuration selon l'invention peut aussi être utilisé pour une présulfuration « ex-situ ».

L'incorporation du soufre dans le catalyseur est effectuée par mise en contact du catalyseur en l'absence d'hydrogène et permet d'obtenir avec une grande précision le degré de sulfuration attendu. Cette incorporation est en général effectuée à une température comprise entre 0 et 50 °C, de préférence entre 0 et 30 °C et avantageusement à température ambiante.

L'agent de sulfuration est en général mis en oeuvre dilué dans un solvant adéquat qui dépend notamment de la nature de l'agent de sulfuration.

Le solvant peut être choisi parmi les solvants suivants, seuls ou en mélange :
essence légère bouillant entre environ 60 et 95 °C,
essence de type hexane bouillant entre environ 63 et 68 °C,
essence de type F bouillant entre environ 100 et 160 °C, et contenant généralement 10 à 20 % d'hydrocarbures aromatiques,
essence de type white spirit bouillant entre environ 150 et 250 °C, et contenant généralement 14 à 22 % d'hydrocarbures aromatiques,
toute coupe hydrocarbonée ou non, équivalente aux essences précédentes.

Dans les exemples ci-dessous, sont indiquées les températures de décomposition du DEDS et du DMDS mis en oeuvre dans un procédé de sulfuration de catalyseur et sont mesurés les dépôt solides, notamment liés au soufre, générés par la présence de DEDS selon l'invention ou d'un polysulfure classiquement utilisé comme agent de sulfuration (ditertiobutylpolysulfure).

L'exemple 3 montre l'amélioration de l'activité des catalyseurs d'hydrotraitement par sulfuration avec du DEDS additivé d'une base parfumante par rapport à une sulfuration avec du DMDS additivé de la même base parfumante.

### Exemple 1

40 cm³ d'un catalyseur commercial d'hydrotraitement de type CoMo sous forme oxyde sont introduits dans un réacteur équipé d'une sonde de température permettant de connaître la température au sein du catalyseur. Le réacteur est placé dans un four permettant de balayer une large gamme de température, pouvant aller jusqu'à 300°C.

Un gasoil non désulfuré de type SRGO (Straight run Gas oil) est additivé de 0,8% en soufre apporté par un agent sulfurant, soit DMDS ou DEDS. Le gasoil additivé est ensuite introduit à température ambiante avec un débit de 80 cm³/h sous un débit d'hydrogène de 20 L/h. Ensuite la température du four est réglée afin d'atteindre 150 °C dans le lit de catalyseur. Le débit de gasoil et d'hydrogène restent fixés respectivement à 80 cm³/h et 20 L/h. Les effluents gazeux en sortie de réacteur sont analysés par chromatographie afin de suivre l'évolution de la concentration en H₂S, représentatif de la décomposition de l'agent de sulfuration. Une fois une valeur stable de concentration en H₂S obtenu à 150 °C, soit [H₂S]_{T=150°C}, la température du lit de catalyseur est augmentée de 10 °C afin de déterminer la concentration à l'équilibre en H₂S à 160 °C, soit [H2S]_{T=160°C}. Cette opération est répétée en augmentant la température de 10 °C en 10 °C jusqu'à atteindre des températures où la concentration en H₂S reste stable et maximale, soit [H₂S]ₘₐₓ. A chaque température intermédiaire est donc connue la concentration en H₂S, soit [H₂S]_{T}. Le taux de décomposition de l'agent sulfurant à une température T est exprimé en % comme le rapport [H₂S]_{T}/[H₂S]ₘₐₓx100.

Le tableau ci-dessous regroupe les taux de décomposition du dimethyldisulfure et du diethyldisulfure :

| Température (°C) | 150 | 200 | 240 | 250 |
|---|---|---|---|---|
| Taux de décomposition de l'agent de sulfuration = DMDS (%) | 0 | 31 | 66 | 100 |
| Taux de décomposition de l'agent de sulfuration = DEDS (%) | 2,5 | 53 | 99 | 100 |

On constate que le DEDS se décompose à plus basse température que le DMDS.

### Exemple 2

Un tube en Incolloy 800HT de 30 cm de long et 7,7cm de diamètre est placé dans un four afin de faire varier la température à l'intérieur du tube entre 200 et 400 °C. Un agent de sulfuration est introduit dans le tube en mélange avec de l'azote afin de respecter un débit d'injection de soufre de 0,8g soufre/h et un débit d'azote de 4 L/h. L'agent de sulfuration est ainsi introduit pendant 3 heures. A la sortie du tube, les effluents sont condensés et récupérés. Après 3 heures d'injection, les condensats récupérés sont filtrés et, le cas échéant, le solide récupéré est pesé.

Les différents agents sulfurant testés sont le DMDS, le DEDS, le DPDS, le DBDS et un ditertiobutyl polysulfure commercialisé par Arkema sous la dénomination TPS54.

Les résultats obtenus sont présentés dans le tableau ci-dessous :

| Agent de Sulfuration | TPS54 | DMDS | DEDS | DPDS | DBDS |
|---|---|---|---|---|---|
| Température (°C) | 350 | 350 | 350 | 350 | 350 |
| Dépôt solide (g) | 1,8 | 0 | 0 | 0 | 0 |

A la vue de ces résultats il apparaît que ni le DEDS, le DPDS et le DBDS ne forment de dépôt solide contrairement au TPS54. Ces mesures confirment la formation possible de dépôt solide observée industriellement lorsque des polysulfures sont mis en oeuvre comme agent de sulfuration.

### Exemple 3

Afin de comparer l'activité du DMDS additivé de 3000 ppm d'un agent masquant d'odeur à celle du DEDS additivé de 3.000 ppm du même agent masquant, un test d'activité a été réalisé sur un pilote d'hydrotraitement. Ce pilote représente une unité industrielle d'hydrotraitement. Le réacteur est chargé avec un catalyseur commercial de type Nickel Molybdène (NiMo) supporté sur alumine. Le réacteur a un volume de 300mL, un diamètre de 17,4 mm et une hauteur de 1.300 mm. Le catalyseur est chargé entre deux couches de carborandum, matériau silico carboné inerte assurant une meilleure distribution des fluides. Le volume de catalyseur chargé est de 20mL. La charge de sulfuration utilisée est un gazole issu de la distillation atmosphérique du pétrole brut (Straight Run Gas Oil : SRGO) additivé de 1% de soufre venant de l'agent de sulfuration. Comme le DMDS et le DEDS n'ont pas la même teneur en soufre, cette procédure permet de comparer des choses comparables. La sulfuration a été conduite sous une pression de 4,5 MPa, une vitesse volumique horaire (VVH) de 1h⁻¹ et un ratio H₂/hydrocarbure(HC) de 200NL/L de la façon suivante :
- Montée en température de 150°C à 230°C à raison de 25°C/h sous charge de sulfuration.
- Suivi en continu de la teneur en H₂S et autre mercaptans dans les gaz sortants du réacteur .
- Palier de température à 230 °C maintenu pendant 4 h (permettant d'atteindre une percée en H₂S supérieure à 3000 ppm).
- Montée en température de 230°C à 350 °C à raison de 25°C/h.
- Palier de 12 h à 350°C.
- Arrêt de la charge de sulfuration et basculement sur la charge de test.

La charge de test utilisée est un mélange de Straight Run Gas Oil (SRGO) et de Light Crude Oil (LCO) dans un ratio 70/30 ayant une teneur en soufre total de 10.400 ppm.

Le test de désulfuration est ensuite réalisé à différentes température (340°C, 350°C et 360°C) avec des périodes de stabilisation afin de connaître l'efficacité du catalyseur sulfuré.

La teneur en soufre total dans la charge après passage dans le réacteur d'hydrotraitement est déterminée en continu lors du test de désulfuration.

L'agent masquant d'odeur a la composition pondérale suivante : acétate d'isoamyle (25 %), orthophtalate de diéthyle (50 %), butyrate de 2-méthylbutyle (15 %) acétate de benzyle (10 %)

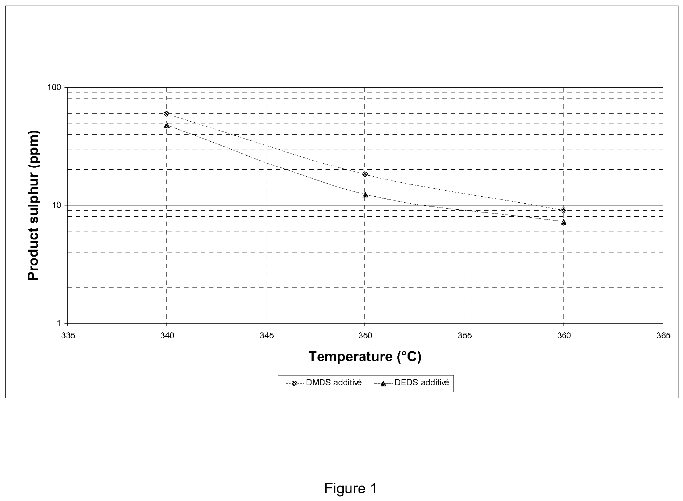

Les résultats sont présentés à la Figure 1:
On constate que le DEDS additivé permet d'obtenir le même niveau de désulfuration (10ppm) que le DMDS additivé mais avec 5 °C de moins (353°C au lieu de 358°C), ce qui est très significatif pour une unité d'hydrotraitement.

## Revendications

1. Agent de sulfuration de catalyseur d'hydrotraitement renfermant un support à base d'au moins un oxyde d'un métal ou d'un métalloïde et au moins un métal actif, **caractérisé en ce qu'**il consiste essentiellement en un disulfure choisi parmi le diéthyldisulfure (DEDS), le(s) dipropyldisulfure(s) (DPDS) ou le(s) dibutyldisulfure(s) (DBDS), et **en ce qu'**il contient moins de 20.000 ppm de traces d'un ou plusieurs sulfures de formule RSnR' avec n représentant le rang moyen en soufre allant de 1 à 10, et R et R' représentant H ou un chaîne alkyle, cycloalkyle, aryle, linéaire ou ramifiée en C₁-C₂₄.

2. Agent de sulfuration selon la revendication 1, **caractérisé en ce qu'**il comprend jusqu'à 10.000 ppm et avantageusement jusqu'à 5.000 ppm desdites traces.

3. Agent de sulfuration selon l'une quelconque des revendications 1 ou 2, **caractérisé en ce qu'**il comprend au moins un agent masquant d'odeur et/ou une base parfumante.

4. Agent de sulfuration selon l'une quelconque des revendications 1 à 3, **caractérisé en ce qu'**il comprend jusqu'à 1 % en poids d'au moins un agent masquant d'odeur et/ou une base parfumante du poids total de l'agent de sulfuration , de préférence de 0,1 à 0,5 %.

5. Procédé de présulfuration de catalyseur renfermant un support à base d'au moins un oxyde d'un métal ou d'un métalloïde et au moins un métal actif, **caractérisé en ce que** l'agent de sulfuration est tel que défini dans l'une quelconque des revendication 1 à 4.

6. Procédé de présulfuration de catalyseur selon la revendication 5 dans lequel la présulfuration est conduite de manière « ex-situ ».

7. Procédé de présulfuration de catalyseur selon la revendication 5 dans lequel la présulfuration est conduite de manière « in-situ ».

## Patentansprüche

1. Mittel zur Sulfidierung eines Hydrotreating-Katalysators, der einen Träger auf Basis von mindestens einem Oxid eines Metalls oder eines Metalloids und mindestens ein aktives Metall enthält, **dadurch gekennzeichnet**, das es im Wesentlichen aus einem Disulfid besteht, dass aus Diethyldisulfid (DEDS), Dipropyldisulfid(en) (DPDS) oder Dibutyldisulfid(en) (DBDS) ausgewählt ist, und dass es weniger als 20.000 ppm Spuren eines oder mehrerer Sulfide der Formel RSnR' enthält, wobei n für den mittleren Schwefelwert im Bereich von 1 bis 10 steht und R und R' für H oder eine lineare oder verzweigte Alkyl-, Cycloalkyl- oder Arylkette mit 1 bis 24 C-Atomen steht.

2. Sulfidierungsmittel nach Anspruch 1, **dadurch gekennzeichnet, dass** es bis zu 10.000 ppm und vorteilhafterweise bis zu 5000 ppm der Spuren enthält.

3. Sulfidierungsmittel nach Anspruch 1 oder 2, **dadurch gekennzeichnet, dass** es mindestens ein geruchsmaskierendes Mittel und/oder eine Parfümierungsbasis umfasst.

4. Sulfidierungsmittel nach einem der Ansprüche 1 bis 3, **dadurch gekennzeichnet, dass** es bis zu 1 Gew.-% mindestens eines geruchsmaskierenden Mittels und/oder einer Parfümierungsbasis, bezogen auf das Gesamtgewicht des Sulfidierungsmittels, und vorzugsweise 0,1 bis 0,5 Gew.-%, umfasst.

5. Verfahren zur Vorsulfidierung eines Katalysators, der einen Träger auf Basis von mindestens einem Oxid eines Metalls oder eines Metalloids und mindestens ein aktives Metall enthält, **dadurch gekennzeichnet, dass** das Sulfidierungsmittel wie in einem der Ansprüche 1 bis 4 definiert ist.

6. Verfahren zur Vorsulfidierung eines Katalysators nach Anspruch 5, bei dem die Vorsulfidierung auf "ex-situ"-Weise durchgeführt wird.

7. Verfahren zur Vorsulfidierung eines Katalysators nach Anspruch 5, bei dem die Vorsulfidierung auf "in-situ"-Weise durchgeführt wird.

## Claims

1. Hydrotreating catalyst sulphiding agent, the catalyst including a support based on at least one oxide of a metal or of a semimetal and at least one active metal, **characterized in that** the sulphiding agent consists essentially of a disulphide chosen from diethyl disulphide (DEDS), dipropyl disulphide(s) (DPDS) and dibutyl disulphide(s) (DBDS), and **in that** it comprises less than 20 000 ppm of traces of one or more sulphides of formula RSnR' with n representing the mean sulphur value ranging from 1 to 10 and R and R' representing H or a C₁-C₂₄ linear or branched, aryl, cycloalkyl or alkyl chain.

2. Sulphiding agent according to Claim 1, **characterized in that** it comprises up to 10 000 ppm and advantageously up to 5000 ppm of said traces.

3. Sulphiding agent according to either of Claims 1 and 2, **characterized in that** it comprises at least one odour-masking agent and/or one scenting base.

4. Sulphiding agent according to any one of Claims 1 to 3, **characterized in that** it comprises up to 1% by weight of at least one odour-masking agent and/or one scenting base of the total weight of the sulphiding agent, preferably from 0.1 to 0.5%.

5. Catalyst presulphidation process, the catalyst including a support based on at least one oxide of a metal or of a semimetal and at least one active metal, **characterized in that** the sulphiding agent is as defined in any one of Claims 1 to 4.

6. Catalyst presulphidation process according to Claim 5, in which the presulphidation is carried out in an "ex situ" way.

7. Catalyst presulphidation process according to Claim 5, in which the presulphidation is carried out in an "in situ" way.
